# EUROPÄISCHE PATENTSCHRIFT

(11) **EP 0 815 830 B1**
(45) Veröffentlichungstag und Bekanntmachung des Hinweises auf die Patenterteilung: **13.12.2000**
(21) Anmeldenummer: 97109824.9
(22) Anmeldetag: 17.06.1997
(51) Int. Cl.: A61K 7/135

(54) **Mittel zum Blondieren von menschlichen Haaren**
Composition for bleaching human hair
Composition pour blanchir des cheveux humains

(30) Priorität: 27.06.1996 DE 19625781; 19.04.1997 DE 19716494
(43) Veröffentlichungstag der Anmeldung: 07.01.1998
(73) Patentinhaber: GOLDWELL GmbH, D-64297 Darmstadt (DE)
(72) Erfinder: Eberling, Walter, 64560 Riedstadt-Crumstadt (DE); Golinski, Frank, Dr., 64297 Darmstadt (DE)

(56) Entgegenhaltungen:
- EP-A- 0 619 113
- EP-A- 0 659 400
- DE-A- 1 617 829
- DE-U- 29 611 217

## Beschreibung

Die vorliegende Erfindung betrifft ein Blondiermittel für menschliche Haare, vorzugsweise in Pulverform, das verbesserte Eigenschaften aufweist.

Blondierpulver sind seit langem bekannt und im Einsatz. Sie enthalten in aller Regel eine pulverförmige Grundlage mit verschiedenen Zusätzen, insbesondere Alkalisierungsmitteln, und als Aktivsubstanz ein Peroxid, in der Regel ein Persulfat, insbesondere Ammoniumpersulfat, gegebenenfalls im Gemisch mit Kaliumpersulfat. Diese Pulver werden bei der Anwendung mit wäßriger, vorzugsweise 6%iger Wasserstoffperoxid-Lösung vermischt.

Es hat sich jedoch gezeigt, daß die Verwendung von Ammoniumpersulfat bei bestimmten Personen zu Allergien führen und auch eine Haarschädigung hervorrufen kann. Es wurde deshalb bereits versucht, persulfatfreie Blondiermittel zu verwenden und durch Erhöhung der Wasserstoffperoxidmenge die gleiche Konzentration an reagiblem Sauerstoff zur Verfügung zu stellen. Dabei wurde jedoch überraschenderweise festgestellt, daß durch diese Maßnahme eine qualitativ gleichwertige Aufhellung des Haares, wie sie mit Ammoniumpersulfat enthaltenden Produkten in Kombination mit Wasserstoffperoxid erhalten wird, mit persulfatfreien Produkten nicht erzielt werden kann.

Es bestand daher die Aufgabe, ein Mittel zum Blondieren von menschlichen Haaren, insbesondere ein Blondierpulver, zu schaffen, das in Kombination mit Wasserstoffperoxid voll wirksam ist, jedoch die mit konventionellen, Ammoniumpersulfat enthaltenden Produkten beobachteten Probleme nicht hervorruft.

Die Lösung dieser Aufgabe besteht darin, ein solches Blondiermittel, insbesondere ein Blondierpulver, zur Verfügung zu stellen, das weitgehend frei von Ammoniumpersulfat ist und trotzdem, in Kombination mit Wasserstoffperoxid, eine ausgezeichnete Blondierwirkung ergibt und ein Gemisch aus Natriumpersulfat und Kaliumpersulfat im Gewichtsverhältnis von 1 zu 9 bis 1 zu 15 enthält.

Vorzugsweise ist die Zusammensetzung so eingestellt, daß beim Mischen mit wäßriger Wasserstoffperoxid-Lösung ein pH-Wert der gebrauchsfertigen Mischung von etwa 9 bis etwa 11, insbesondere zwischen 9,5 bis 10,5, erhalten wird.

Gegenstand der Erfindung ist daher auch ein Verfahren zum Blondieren von menschlichen Haaren, wobei das erfindungsgemäße Mittel unmittelbar vor dem Aufbringen auf das Haar mit einer wäßrigen, Wasserstoffperoxid enthaltenden Zusammensetzung gemischt wird und die erhaltene Mischung einen pH-Wert zwischen 9 und 11, insbesondere von 9,5 bis 10,5 aufweist.

Es hat sich nämlich gezeigt, daß bei der Einstellung eines solchen pH-Wertes einerseits eine optimale Blondierwirkung und andererseits eine Haarschonung erzielt werden kann.

Die Mitverwendung weiterer Peroxide in untergeordneten Mengen, z. B. Magnesiumperoxid, Melaminperoxid oder Harnstoffperoxid, ist im Prinzip möglich; der Anteil an Ammoniumpersulfat sollte 0 bis maximal 1 Gew.-% betragen.

Gemäß einer bevorzugten Ausführungsform der Erfindung können die Blondiermittel noch 0,1 bis 10 Gew.-%, berechnet auf die Gesamtzusammensetzung, eines oder mehrerer Ammoniumsalze enthalten.

Geeignete Ammoniumsalze sind dabei Ammoniumcarbonat, Ammoniumhydrogencarbonat, Ammoniumcarbamat, Ammoniumchlorid, Ammoniumsulfat, Ammoniumphosphate, Ammoniumnitrat, Ammoniumbromid, Ammoniumjodid, Ammoniumthiosulfat, Ammoniummolybdat, Ammoniumvanadat, Ammoniumsulfamat, Ammoniumcitrat, Ammoniumsalicylat, Ammoniumvalerat, Ammoniumtartrat, Ammoniumbenzoat, Ammoniumacetat, Ammoniumformiat und Ammoniumlactat.

Bevorzugt sind hierbei die Ammoniumphosphate wie NH₄H₂PO₄, (NH₄)₂HPO₄, (NH₄)₂NaPO₄, NaNH₄HPO₄ oder NH₄Na₂PO₄, Ammoniumchlorid, Ammoniumsulfat und Diammoniumhydrogencitrat sowie Ammoniumchlorid, vorzugsweise in einer Menge von 0,1 bis 5 Gew.-% Ammoniumchlorid.

Neben dem Natriumpersulfat/Kaliumpersulfat-Gemisch enthalten die erfindungsgemäßen Blondiermittel mit verringerter Haarschädigung und verbesserter Blondierwirkung die in solchen Mitteln üblichen Bestandteile:

Wenn es sich um ein Pulver handelt, insbesondere inerte pulverförmige Trägerstoffe, beispielsweise pyrogenes Siliciumdioxid, Stärkepulver, etc., Alkalisierungsmittel wie Natriummetasilikat, oberflächenaktive Substanzen, Bindemittel, etc.; zur Vermeidung von Wiederholungen wird auf die einschlägigen Standardwerke, beispielsweise K.Schrader, "Grundlagen und Rezepturen der Kosmetika", 2. Auflage (1989, Hüthig Buchverlag), S.815 bis 823, verwiesen.

Diese Pulver können auch, wie aus der EP-A 560 088 bekannt, mit Ölen bzw. mit einem flüssigen Wachs beschichtet sein oder, wie es in der EP-A 630 643 beschrieben ist, als mit Binde- und Verdickungsmitteln verfestigtes Agglomerat vorliegen und dadurch staubfrei gemacht werden.

Obwohl die bevorzugte Ausführungsform des erfindungsgemäßen Mittels ein wasserfreies Blondierpulver darstellt, ist es auch möglich, andere Trägermaterialien zu verwenden, beispielsweise wasserfreie Suspensionen oder Pasten, wie sie z.B. in der DE-A 38 14 356 beschrieben sind.

Im folgenden wird die überraschende Wirkung der erfindungsgemäßen Blondiermittel anhand von einem Beispiel illustriert.

Die erfindungsgemäßen Blondiermittel werden vor ihrer Anwendung auf dem Haar, wie bereits oben ausgeführt, mit einer wäßrigen, Wasserstoffperoxid enthaltenden Zubereitung angemischt, vorzugsweise im Verhältnis 1 zu 1 bis 1 zu 2, berechnet auf eine 6%ige H₂O₂-Zusammensetzung.

Aus der DE 16 17 829 sind Mittel zum Blondieren von lebenden Haaren bekannt, die neben Ammoniumpersulfat, gleiche Mengen an Natrium- und Kaliumpersulfat sowie Reduktionsmittel enthalten. Dadurch sollen der Blondiereffekt und insbesondere die Lagerstabilität solcher Mittel verbessert werden.

Die EP-A 659 400 beschreibt ein Bleichverfahren für Keratinfasern, das eine ein Oxidationsmittel enthaltende Zusammensetzung bei Temperaturen von mindestens 75 °C zusammen mit Wasserdampf anwendet.

Die EP-A 619 113 schließlich offenbart staubfreie peroxidhaltige Blondiermittel, die mit Polypropylenglykol mit einem Molekulargewicht von 200 bis 30.000 granuliert sind.

### Blondierpulver

### Bestandteile in Gewichtsteilen

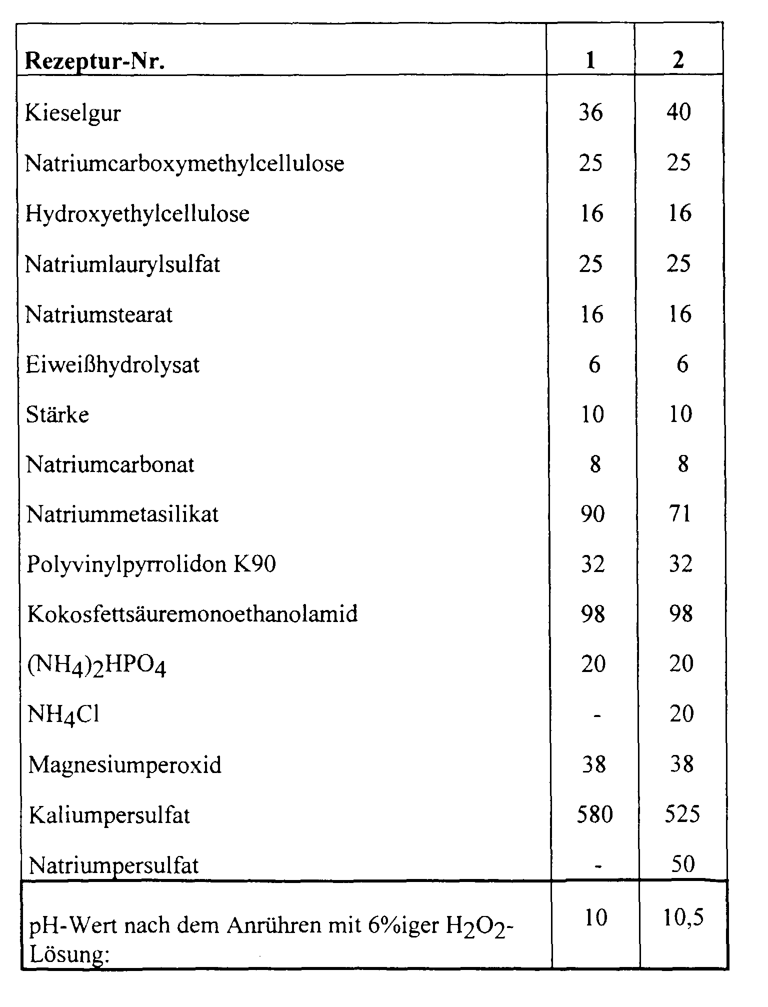

25 g der Blondierpulver-Zusammensetzungen Nr. 1 und 2 wurden jeweils mit 35 g 6%iger wäßriger, stabilisierter Wasserstoffperoxid-Lösung angerührt, wobei die in der vorgenannten Tabelle angegebenen pH-Werte erhalten wurden.

Diese Zusammensetzungen wurden auf Strähnen aus dunkelbraunem Menschenhaar aufgebracht. Nach 15minütiger Einwirkung bei 50 °C wurden die Strähnen gewaschen, getrocknet und die erzielte Aufhellung mittels eines "Minolta Chroma-Meters C 200" nach der bekannten CIE-L^{x}a^{x}b^{x}-Methode nach Judd und Hunter bestimmt.

Dabei wurden die folgenden Aufhellungswerte L erhalten:

| **Zusammensetzung Nr.** | **Aufhellung L** |
|---|---|
| Unbehandeltes Haar | 24,14 |
| 1 | 55,90 |
| 2 | 60,30 |

Diese Werte zeigen eindrucksvoll die durch die erfindungsgemäße Zusammensetzung erzielte verbesserte Blondierwirkung.

## Patentansprüche

1. Mittel zum Blondieren von menschlichen Haaren, das maximal 1 Gew.-% Ammoniumpersulfat aufweist, enthaltend ein Gemisch aus Natriumpersulfat und Kaliumpersulfat im Gewichtsverhältnis von 1 zu 9 bis 1 zu 15.

2. Mittel nach Anspruch 1, enthaltend 0,05 bis 10 Gew.-% eines Ammoniumsalzes, ausgenommen Ammoniumpersulfat.

3. Mittel nach Anspruch 2, enthaltend 0,1 bis 5 Gew.-% Ammoniumchlorid.

4. Verfahren zum Blondieren von menschlichen Haaren, wobei unmittelbar vor der Blondierung ein Mittel nach einem oder mehreren der Ansprüche 1 bis 3 mit einer wäßrigen, Wasserstoffperoxid enthaltenden Zusammensetzung vermischt und die erhaltene Mischung, die einen pH-Wert von 9 bis 11 aufweist, auf das Haar aufgebracht wird.

## Claims

1. Composition for bleaching of human hair, containing not more than 1% by weight ammonium persulfate, comprising a mixture of sodium persulfate and potassium persulfate in a weight ratio from 1:9 to 1:15.

2. Composition according to claim 1, comprising 0.05% to 10% by wt. of an ammonium salt, excluding ammonium persulfate.

3. Composition according to claim 2, comprising 0.1% to 5% by wt. ammonium chloride.

4. Process for bleaching of human hair, wherein a composition according to one or more of the claims 1 to 3 is mixed with an aqueous hydrogen peroxide composition prior to use, whereupon the mixture achieved, having a pH-value from 9 to 11, is applied onto the hair.

## Revendications

1. Composition pour blondir les cheveux humains contenant au maximum 1 % en poids de persulfate d'ammonium, se composant d'un mélange de persulfate de sodium et de persulfate de potassium selon un rapport pondéral de 1 pour 9 à 1 pour 15.

2. Composition selon la revendication 1, contenant 0,05 à 10 % en poids d'un sel d'ammonium, abstraction faite du persulfate d'ammonium.

3. Composition selon la revendication 2, contenant 0,1 à 5 % en poids de chlorure d'ammonium.

4. Procédé pour blondir les cheveux humains, dans lequel on mélange, immédiatement avant la décoloration, une composition selon une ou plusieurs des revendications 1 à 3, avec une composition aqueuse contenant du peroxyde d'hydrogène, et dans lequel on applique sur la chevelure le mélange obtenu, qui présente un pH entre 9 et 11.
